# FASCICULE DE BREVET EUROPEEN

(11) **EP 4 559 419 B1**
(45) Date de publication et mention de la délivrance du brevet: **04.11.2026**
(21) Numéro de dépôt: 25163165.1
(22) Date de dépôt: 01.04.2014
(51) Int. Cl.: A61M 25/00, A61B 17/3203, A61B 18/14, A61B 18/00, A61B 17/00

(54) **CATHETER BI-FONCTIONNEL A DEUX GAINES COULISSANTES**
BIFUNKTIONELLER KATHETER MIT ZWEI SCHIEBESCHLÄUCHEN
DUAL-FUNCTION CATHETER WITH DUAL SLIDING SHEATHS

(30) Priorité: 04.04.2013 FR 1353033
(43) Date de publication de la demande: 28.05.2025
(62) Numéro(s) de document de la (des) demande(s) initiale(s) en application de l'article 76 CBE: 21175191.2 / 3 906 960; 14722250.9 / 2 981 321
(73) Titulaire: Boston Scientific Medical Device Limited, Galway (IE)
(72) Inventeur: Gonon, Bertrand, décédé(e) (FR)
(74) Mandataire: Vossius & Partner Patentanwälte Rechtsanwälte mbB

(56) Documents cités:
- EP-A1- 1 462 142
- EP-A1- 1 800 612
- WO-A1-2011/148333
- FR-A1- 2 830 455
- US-A1- 2011 028 887
- US-A1- 2011 092 892
- US-A1- 2012 035 607

## Description

La présente invention concerne un cathéter, et plus particulièrement un cathéter d'injection de fluide.

Traditionnellement, un cathéter d'injection de fluide comprend une gaine avec une buse d'injection, reliée par ailleurs à un réservoir de fluide 10 permettant, par exemple d'injecter un fluide sous pression.

On connait par exemple le document FR2830455 qui décrit un cathéter pour injecter un fluide sous pression. Le cathéter comprend une gaine avec un outil d'injection rétractable à son extrémité distale, l'outil d'injection étant perforant, coupant, ou piquant.

Or dans un tel cathéter, l'injection de fluide commande la sortie de l'outil, ce qui, bien que présentant l'avantage de pouvoir escamoter automatiquement l'outil perforant, coupant, ou piquant, requiert un minimum de pression du fluide afin de pouvoir faire sortir l'outil. De plus, certaines applications peuvent requérir un positionnement plus précis de l'outil avant d'injecter le fluide. Cependant, il est préférable de protéger les organes ou les tissus de tout élément coupant, comme l'outil par exemple. Il est ainsi plus prudent d'insérer un cathéter comprenant un outil coupant avec l'outil protégé. La présente invention vise à remédier à au moins une partie des inconvénients précités et à conduire en outre à d'autres avantages.

EP1 800 612 A1 décrit un instrument chirurgical ayant une buse d'injection et une électrode, les deux éléments étant séparés. US201 2/035607 A1 décrit un instrument chirurgical endoscopique ayant une gaine extérieure coulissant sur une gaine intérieure. EP 1 462 142 A1 décrit un cathéter avec capteur de ponction.

L'invention est définie par un cathéter selon la revendication 1. D'autres aspects sont définis dans les revendications 2-11.

Un tel cathéter permet ainsi une indépendance entre une injection de fluide et une rétractation de la buse, de manière simple et sécuritaire, notamment si la buse est piquante ou coupante. Dans un cas général, une buse étant typiquement très fine de géométrie, elle est ainsi protégée lors de l'insertion du cathéter pour éviter qu'elle ne soit abimée accidentellement, ou qu'elle ne griffe ou accroche un tissu ou un organe lors de l'insertion du cathéter.

On entend ici par « fluide » tout liquide, comme de l'eau, une solution saline ou un liquide présentant une viscosité importante, ainsi que des macromolécules ou des molécules thérapeutiques.

En effet, le fluide sous pression est par exemple une solution saline ou une solution plus visqueuse selon les nécessités de l'application. Injecter un fluide visqueux permet notamment une résorption dans un tissu plus lente que si le fluide est très peu visqueux.

La buse d'un tel cathéter présente alors deux positions : une position escamotée dans laquelle elle est recouverte au moins en partie par la gaine extérieure, et une position sortie, proéminente, pour une utilisation précise.

La gaine extérieure, coulissant sur la gaine intérieure, est par exemple plus courte que la gaine intérieure.

En d'autres termes, la buse, la gaine intérieure et le connecteur forment un premier ensemble, et la gaine extérieure et le manchon forment un second ensemble, apte à coulisser par rapport au premier ensemble.

On désigne ici par « manchon » tout élément ou partie, optionnellement texturée, qui facilite un agrippement à la gaine extérieure pour lui induire un mouvement, poussé ou tiré. Ce peut être une zone texturée dans la gaine ou une bague en plastique par exemple.

Dans la position poussée, le manchon est par exemple éloigné du connecteur par rapport à la position tirée dans laquelle il est par exemple rapproché du connecteur.

Ainsi, lorsque le cathéter est introduit, par exemple dans un organe, la buse, escamotée au moins en partie, permet une insertion plus sure, c'est-à-dire avec moins de risque de perforer, ou griffer, accidentellement une partie de l'organe ou un autre organe par exemple. Une fois une extrémité distale du cathéter en position, la buse est sortie par une traction de la gaine extérieure, via le manchon, et il est alors possible d'ajuster sa position si nécessaire.

On entend ici par organe tout organe, tissu, veine ou artère, ou toute partie du corps pour laquelle un tel cathéter serait nécessaire pour au moins une injection de fluide.

Le fait que ce soit la gaine extérieure qui est mobile facilite toute réalisation de liaison étanche entre la gaine intérieure dans laquelle s'écoulerait un fluide et un réservoir de fluide ou autres éléments. Ceci permet ainsi de réduire des coûts de réalisation.

La buse pénètre ensuite dans une couche superficielle de l'organe, par exemple une muqueuse. Il est alors possible de soulever et séparer un polype ou une lésion d'une muqueuse par rapport à une couche musculaire sous-jacente, pour ensuite retirer le polype ou la lésion par exemple. L'injection du fluide sépare la muqueuse du muscle et soulève le polype situé dans la muqueuse, qui est ensuite possiblement coupé et disséqué par le fluide sous haute pression et optionnellement aidé d'une électrode raccordée à un générateur électrique (par exemple une ESU, pour « electrical surgical unit » en anglais, i.e. une unité d'électro chirurgie, pour chirurgie endoscopique).

La buse d'injection est optionnellement perforante pour faciliter l'injection d'un fluide sous une membrane ou une muqueuse recouvrant une couche musculaire par exemple.

Les gaines intérieure et extérieure présentent de préférence une souplesse, une flexibilité, selon leur longueur, afin de pouvoir être facilement introduites dans une veine, une artère ou un quelconque organe et dans un outil médical comme un endoscope. Toutefois, il est préférable de pouvoir éviter de produire un pliage des gaines, et de limiter des déformations irréversibles d'une section des gaines qui obstrueraient le passage du fluide (par exemple un cintrage, en particulier de la gaine intérieure si celle-ci est fléchie). Pour cela, les gaines sont par exemple réalisées dans des polymères biocompatibles.

Selon un exemple de réalisation, la gaine intérieure est en PEBA.

Selon un exemple de réalisation, la gaine extérieure est en téflon.

Un tel cathéter permet de limiter voire éviter des pertes de pression et présente une flexibilité importante sans permettre un pliage facile irréversible naturellement (plasticité).

Grâce à sa souplesse, il permet aussi d'injecter des fluides plus ou moins visqueux en vision directe et en rétroflexion lorsqu'il est inséré, par exemple, dans un endoscope présentant une plasticité lui permettant d'être coudé.

Ainsi, selon un aspect original en soi, indépendamment d'un glissement entre les gaines, est également proposé un cathéter d'injection de fluide comprenant un conduit, caractérisé en ce qu'il comprend une gaine intérieure réalisée en PEBA qui forme le conduit, et une gaine extérieure réalisée en téflon, entourant la gaine intérieure. La gaine intérieure est optionnellement fixée par une extrémité proximale à un connecteur permettant de relier le cathéter à un réservoir de fluide. Une buse d'injection est aussi, par exemple, fixée à une extrémité distale de la gaine intérieure.

Le cathéter comprend une bague d'embout fixée à une extrémité distale de la gaine extérieure apte à coulisser sur au moins une partie de la buse d'injection.

Une telle bague d'embout permet par exemple une continuité de surface avec la buse escamotée au moins en partie pour faciliter l'introduction du cathéter. Elle facilite par ailleurs un guidage entre la gaine intérieure et la gaine extérieure lorsqu'elles coulissent l'une par rapport à l'autre. Enfin, une telle bague d'embout est par exemple isolante, elle permet de contrôler les mouvements de la buse, et sert aussi optionnellement de joint d'étanchéité pour éviter que du fluide ne s'infiltre entre la gaine intérieure et la gaine extérieure.

Par exemple, la bague d'embout est en céramique, car la céramique est un matériau facile à mettre en œuvre (moulage ou usinage par exemple), et à la fois biocompatible et isolant électriquement. D'autres matériaux sont bien entendu envisageables, comme par exemple du polymère.

La bague d'embout présente par exemple une tête de forme distale arrondie. Une telle forme permet une meilleure ergonomie lors de l'introduction du cathéter alors que la buse est escamotée au moins en partie.

Selon un mode intéressant de réalisation, la buse d'injection forme en outre une électrode de sorte que le cathéter est un cathéter bi-fonctionnel.

Un exemple non revendiqué d' un cathéter bi-fonctionnel comprend:
- un conduit,
- un connecteur comprenant une connexion fluidique et une connexion électrique,
- une gaine intérieure, formant le conduit, fixée par une extrémité proximale à une connexion de sortie du connecteur permettant de relier le cathéter à un réservoir de fluide,
- une buse d'injection fixée à une extrémité distale de la gaine intérieure,
- une électrode,
- et une gaine extérieure, entourant la gaine intérieure, fixée à un manchon, le manchon et la gaine extérieure coulissant par rapport à la gaine intérieure et prenant au moins une position poussée (« b ») dans laquelle la gaine extérieure recouvre au moins une partie de la buse d'injection et une position tirée (« a ») dans laquelle la gaine extérieure découvre ladite partie de la buse d'injection,
caractérisé en ce que la buse d'injection forme l'électrode de sorte que la buse et l'électrode sont regroupées en un seul élément.

Un tel cathéter bi-fonctionnel permet ainsi de soulever et détacher un polype ou une lésion d'une couche musculaire inférieure et de retirer le polype ou la lésion avec un seul outil présentant entre autres les avantages précédemment cités, comme par exemple une insertion du cathéter sécurisée puisque la buse (faisant également office d'électrode) est escamotée au moins en partie. La perforation du tissu pour l'injection du fluide peut alors être également réalisée par la buse, ensuite introduite sous le tissu pour l'injection du fluide, qui peut ainsi se dispenser d'une forme biseautée par exemple. Après injection, c'est également la buse qui peut alors découper le polype ou la lésion pour les retirer.

Un cathéter bi-fonctionnel désigne ici un cathéter d'injection et de dissection par électrochirurgie c'est-à-dire un cathéter permettant d'injecter un fluide sous pression sous un tissu ou une muqueuse, et de découper le tissu ou la muqueuse avec une électrode en un même outil. Ainsi, l'électrode considérée ici est une électrode de dissection électrochirurgicale.

Autrement dit, un tel cathéter permet d'injecter un fluide sous pression à travers une buse présente dans une électrode.

Selon un exemple de réalisation privilégié, le jet de fluide passe au centre de l'électrode.

L'électrode, qui est alors également la buse, comporte par exemple un canal central, configuré pour injecter du fluide.

Un jet central est mieux adapté pour injecter du fluide pour gonfler une zone (par exemple détacher la muqueuse du muscle pour plus facilement découper la muqueuse sans risque d'atteindre le muscle), alors que lorsqu'un jet est en périphérie d'une électrode, celui-ci est plus adapté pour un lavage de la zone par exemple. La buse et l'électrode alors regroupées en un seul élément sont ainsi centrées l'une par rapport à l'autre. L'élément formant la buse et l'électrode présente par exemple une symétrie axiale.

Selon un exemple de réalisation, l'électrode comprend un anneau en forme de plateau circulaire troué venant se fixer à une extrémité distale de l'électrode.

Selon un exemple de réalisation, le connecteur comprend une connexion fluidique et une connexion électrique.

Le connecteur est par exemple un connecteur en Y ou un connecteur en T avec une connexion fluidique pour le réservoir de fluide et une connexion électrique, outre une connexion, dite « de sortie », le reliant à la gaine intérieure.

Selon un exemple particulier de réalisation, l'électrode est reliée à la connexion électrique par un fil conducteur courant dans la gaine intérieure. Le fil conducteur est conducteur électriquement, et est par exemple un fil métallique.

En d'autres termes, le cathéter bi-fonctionnel comporte un fil conducteur disposé dans la gaine intérieure et relié d'une part à l'électrode, c'est-à-dire à la buse, et d'autre part à la connexion électrique.

En outre, la gaine intérieure relie le connecteur à l'électrode, qui fait office de buse. Ainsi en pratique, le fluide à injecter circule depuis la connexion fluidique, dans la gaine intérieure, jusque dans l'électrode, grâce à son canal. Le fil conducteur est alors immergé dans le fluide à injecter.

Ainsi, par exemple, contrairement à d'autres dispositifs dans lesquels l'électrode est reliée par un tube métallique tout du long du cathéter, pouvant former en soi le conduit, un tel cathéter a davantage de souplesse, de flexibilité, et permet d'éviter des bouchages (par pliage du tube métallique induisant un ceintrage), des pertes de charge et de pression ou encore un blocage en translation de la gaine extérieure par rapport à la gaine intérieure.

A titre illustratif, il est aussi présenté un dispositif comprenant un générateur de jets pulsés comprenant un réservoir et un cathéter, caractérisé en ce que le cathéter est un cathéter tel que défini précédemment relié d'une part au générateur de jets pulsés par une connexion fluidique du connecteur.

Le dispositif génère, transmet via le cathéter et injecte par la buse, située à une extrémité du cathéter, le fluide sous pression (par exemple une solution saline) dans un tissu mou (comme la muqueuse, ou une sous-muqueuse).

Si le cathéter est bi-fonctionnel, le dispositif comprend alors une buse d'injection et une électrode raccordée à un générateur électrique (une « ESU » pour « electro surgical unit »), l'électrode et la buse étant alors réalisées en un même élément. Le cathéter est alors relié en outre d'autre part à un générateur électrique par une connexion électrique du connecteur.

Selon un exemple intéressant de réalisation, le générateur de jets pulsés est un générateur de jets pulsés haute pression prévu pour générer des impulsions de fluide sous haute pression à partir d'au moins un fluide contenu dans une poche souple formant le réservoir placée dans une enceinte étanche mise sous pression, l'enceinte présentant au moins une sortie pour fluides, le générateur comprenant :
- un dispositif de pressurisation pour l'enceinte qui comprend une source de gaz sous pression qui fournit un gaz sous pression pénétrant dans l'enceinte pour augmenter sa pression interne,
- un dispositif de régulation de la pression qui régule la pression à l'intérieur de l'enceinte,
- au moins un connecteur de jonction d'une poche souple avec un conduit de raccordement, ce connecteur de jonction et le conduit de raccordement étant disposés dans l'enceinte,
- un séquenceur hydraulique comprenant au moins :
   - une entrée dans laquelle arrive au moins le conduit de raccordement, et une sortie à laquelle est reliée un conduit de sortie communiquant hydrauliquement avec l'extérieur du générateur,
   - une enveloppe déformable souple prévue dans une liaison hydraulique, le tout étant exposé à la pression régnant dans l'enceinte,
   - au moins un obturateur hydraulique dans la liaison hydraulique,
   - un moyen de commande des obturateurs hydrauliques,
- et le conduit de sortie étanche entre la sortie du séquenceur et la sortie de l'enceinte raccordée à un conduit de liaison extérieur avec le cathéter.

Un tel générateur est décrit dans la demande de brevet WO 2011/148333.

Par exemple, le générateur est apte à générer un pulse de fluide à une pression comprise entre 0,2 et 3,0 MPa (2 et 30 bars).

La pression du fluide est par exemple de 1,0 MPa (10 bars - soit environ 145 psi) pour une solution saline (~eau), et de 2.0 MPa (20 bars - soit environ 290 psi) lorsque le fluide est visqueux.

Par exemple, le générateur est apte à générer un pulse de fluide d'un volume de fluide par pulse compris entre 100 µL et 10 mL.

Le pulse est contrôlé par une électrovanne tout-ou-rien (souvent nommée « électrovanne clamp » ou « on-off », qui permet de générer des pulses, par opposition à un jet continu par exemple) située dans l'enceinte pressurisée elle-même contrôlée par exemple par une interface-utilisateur. L'électrovanne forme par exemple au moins un obturateur hydraulique située en amont de l'enveloppe déformable souple.

On entend ici par enveloppe déformable souple qu'une partie de la liaison hydraulique se déforme en fonction de variation de pression régnant dans l'enceinte. Elle est par exemple réalisée grâce à une partie de paroi de la liaison hydraulique amincie, ou par un bulbe formé dans la liaison hydraulique. Selon un autre exemple, l'enveloppe déformable souple est aussi optionnellement un élément indépendant, la liaison hydraulique comprenant alors une partie amont et une partie aval par rapport à l'enveloppe déformable souple.

Un tel dispositif permet ainsi de limiter voire éviter des risques de perforation du muscle et a un coût de réalisation faible.

Ainsi un tel dispositif permet de couper et disséquer des tissus mous en chirurgie ouverte, aussi bien que par endoscopie et/ou laparoscopie.

L'invention, selon un exemple de réalisation de l'invention, sera bien comprise et ses avantages apparaitront mieux à la lecture de la description détaillée qui suit, donnée à titre illustratif et nullement limitatif, en référence aux dessins annexés dans lesquels :
La figure 1 représente un cathéter bi-fonctionnel selon un exemple de réalisation de l'invention, la figure 2 représente une vue de dessus, en coupe partielle, du cathéter de la figure 1 ,
la figure 3 présente un exemple de réalisation en coupe du cathéter de la figure 1,
les figures 4 à 15 illustrent des exemples de réalisation de différents éléments constitutifs du cathéter des figures 1 à 3 :

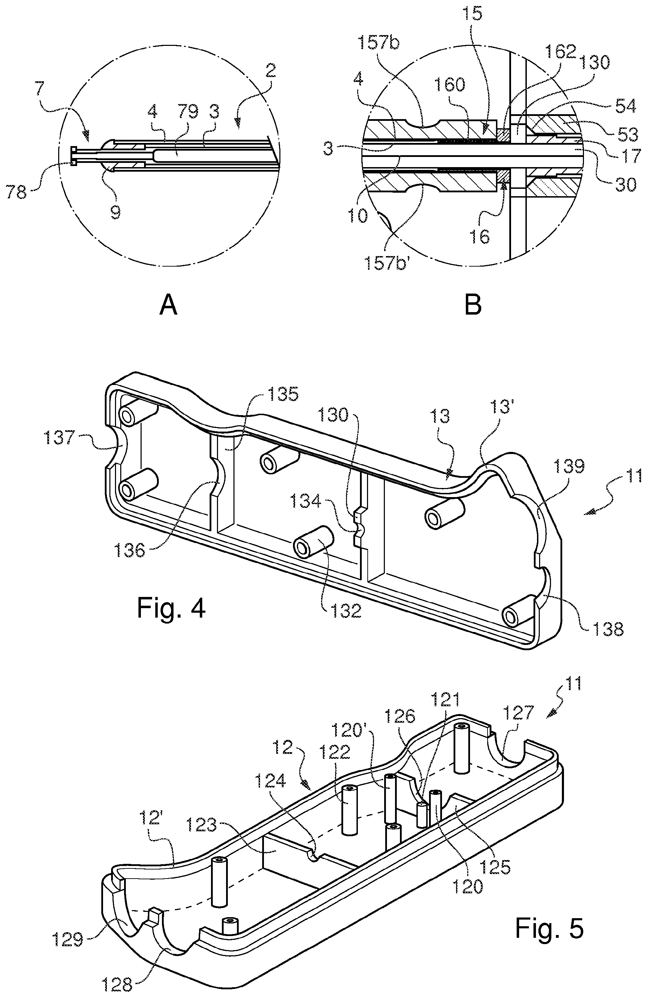

   les figures 4 et 5 représentent un exemple de réalisation d'un boitier en deux parties du cathéter,

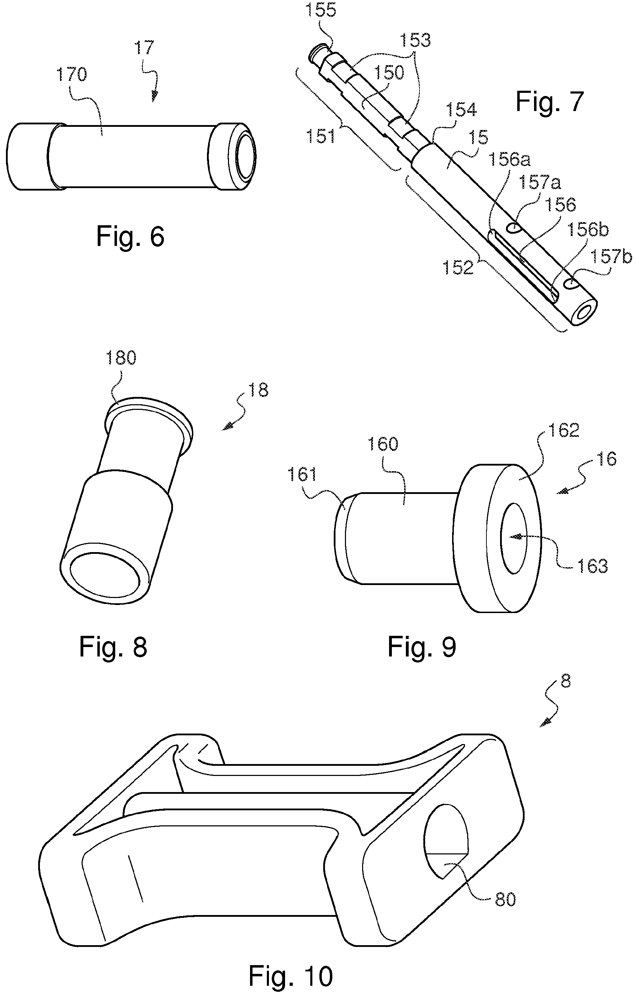

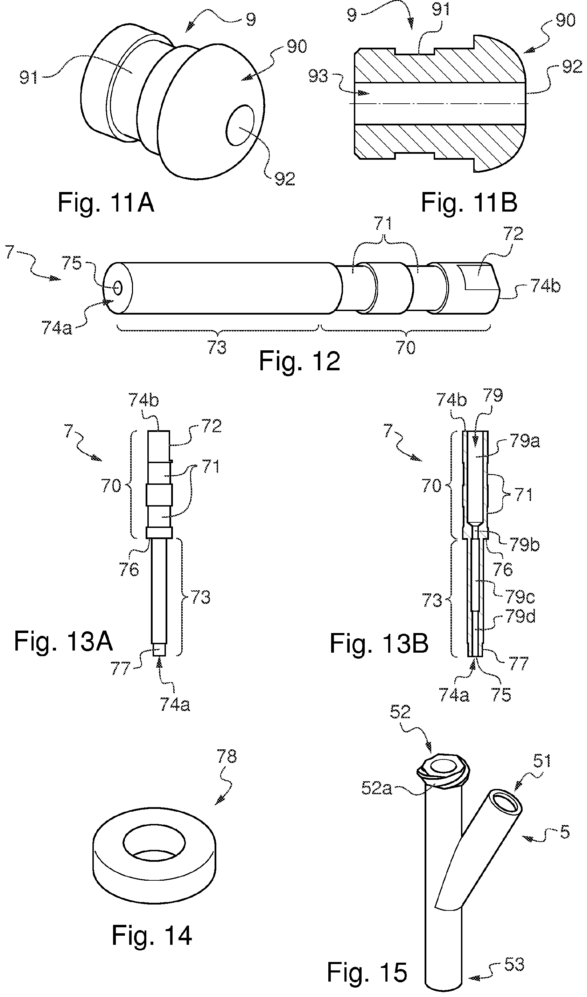

   la figure 6 représente un exemple de réalisation d'un tube permettant de solidariser une gaine intérieure à un connecteur détaillé figure 15,
   la figure 7 représente un exemple de réalisation d'un élément intermédiaire pour relier un manchon représenté figure 10 à une gaine extérieure grâce à une bague illustrée figure 9,
   la figure 8 représente un exemple de réalisation d'un protège fiche électrique,
   la figure 9 représente un exemple de réalisation de la bague permettant de fixer la gaine extérieure à l'élément intermédiaire,
   la figure 10 représente un exemple de réalisation du manchon,
   les figures 11a et 11b représentent respectivement une vue en perspective et une vue en coupe d'une bague d'embout de la gaine extérieure selon un exemple de réalisation de la présente invention,
   la figure 12 représente une buse selon un mode de réalisation de la présente invention,
   les figures 13a et 13b représentent une buse, respectivement en volume et en coupe, selon un autre mode de réalisation de la présente invention,
   la figure 14 représente un anneau pour une extrémité distale d'une buse selon un mode de réalisation de la présente invention, l'anneau ayant une forme de plateau circulaire troué apte à venir se fixer à une extrémité de la buse telle que représentée par exemple figures 13a et 13b,
   la figure 15 illustre un exemple de réalisation d'un connecteur à deux voies d'entrée,

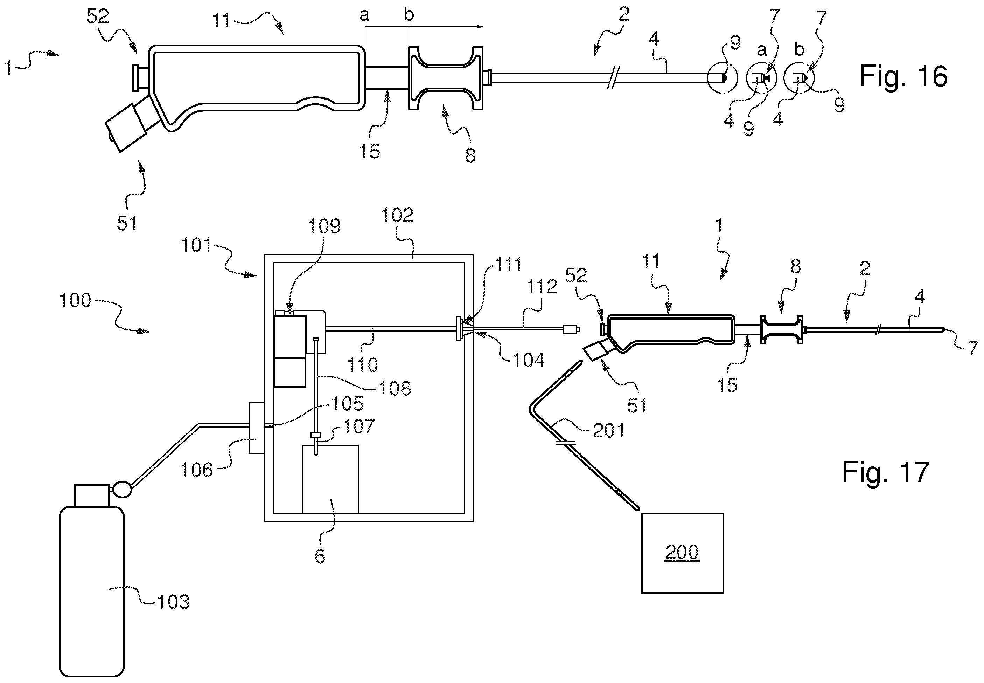

la figure 16 illustre un lien entre la position du manchon et la sortie de la buse selon un exemple de réalisation de la présente invention, et
la figure 17 illustre un dispositif selon un mode de réalisation de l'invention.

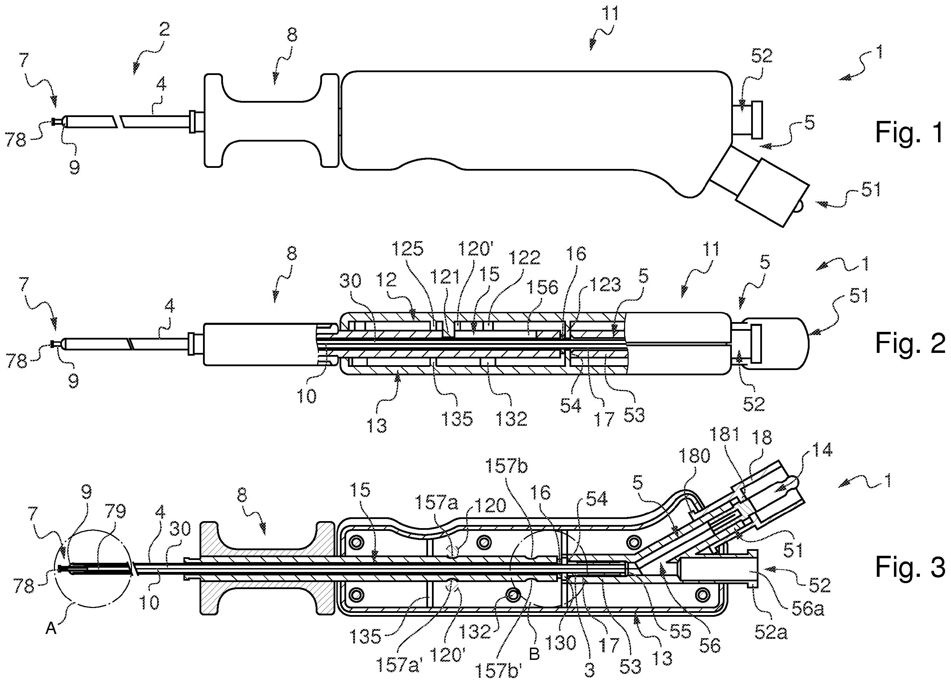

Les éléments identiques représentés sur les figures 1 à 17 sont identifiés par des références numériques identiques.

Dans le présent exemple de réalisation de l'invention, un cathéter 1 d'injection de fluide comprend un conduit 2 relié à un boitier 11 comprenant un connecteur 5.

Le conduit 2 comprend :
- une gaine intérieure 3 (visible par exemple dans le détail A de la figure 3), fixée par une extrémité proximale au connecteur 5 permettant de relier le cathéter 1 à un réservoir de fluide 6 (représenté figure 17),
- une buse d'injection 7 fixée à une extrémité distale de la gaine intérieure 3,
- et une gaine extérieure 4, entourant la gaine intérieure 3, fixée à un manchon 8, le manchon 8 et la gaine extérieure 4 coulissant par rapport à la gaine intérieure 3 et prenant au moins une position poussée (position « b » illustrée figure 16) dans laquelle la gaine extérieure 4 recouvre au moins une partie de la buse d'injection 7, et une position tirée (position « a » illustrée figure 16) dans laquelle la gaine extérieure 4 découvre ladite partie de la buse d'injection 7.

La gaine intérieure 3 est par exemple réalisée en PEBA, et la gaine extérieure 4 est par exemple réalisée en téflon.

Une bague d'embout 9 est ici fixée à une extrémité distale de la gaine extérieure 4 et est apte à coulisser sur au moins une partie de la buse d'injection 7.

La bague d'embout 9 est par exemple en céramique. Elle présente en outre une tête 90 de forme distale arrondie, comme l'illustre les figures 11a et 11b.

En d'autres termes, le cathéter 1 comprend deux ensembles aptes à coulisser l'un par rapport à l'autre.

Un premier ensemble, dit ici « ensemble fixe » comprend la buse 7, la gaine intérieure 3, un tube 17, le connecteur 5, un protège fiche 18 et la fiche électrique 14, et le boitier 11.

Un second ensemble, dit ici « ensemble mobile » comprend la bague d'embout 9, la gaine extérieure 4, le manchon 8, un élément intermédiaire 15, et une bague 16.

Bien entendu, ces définitions d'ensembles servent à fixer les idées dans la présente description, et les attributs « fixe » et « mobile » sont ici arbitraires pour définir un ensemble par rapport à l'autre.

En référence à l'ensemble fixe, comme le montre le détail A de la figure 3, la buse 7 est enfoncée dans la gaine intérieure 3. Elle est par exemple collée à l'intérieure de la gaine intérieure 3 à son extrémité distale.

Comme le montrent les figures 12 à 14, la buse 7 présente ici une zone de liaison 70 favorisant une attache entre la buse 7 et la gaine intérieure 3. La zone de liaison 70 présente en outre ici des gorges 71 et un méplat 72. Les gorges 71 permettent par exemple de servir de déversoir pour de la colle si l'assemblage est réalisé par collage, afin d'éviter que de la colle ne déborde, ce qui procure en outre un rendu peu soigné et inesthétique. Les gorges 71 permettent aussi un meilleur agrippement entre la buse 7 et la gaine intérieure 3, tandis que le méplat 72 sert de détrompeur pour l'insertion de la buse 7 dans la gaine intérieure 3 et/ou de repère s'il est préférable d'insérer la buse 7 selon une certaine orientation. Le méplat 72 permet aussi de faciliter l'insertion de la buse 7 dans la gaine intérieure 3 puisqu'il induit une réduction de section. Selon un autre mode de réalisation non représenté, il pourrait toutefois n'y avoir qu'une seule gorge 71.

La buse 7 présente en outre ici une zone utile 73, hors de la gaine intérieure 3 lorsqu'elles sont assemblées l'une à l'autre. La zone utile 73 présente une extrémité distale 74a présentant un orifice de sortie 75 par lequel est injecté le fluide dans ou sous un tissu le cas échéant. L'extrémité distale 74a est optionnellement piquante ou coupante, ou par exemple biseautée (non représenté ici).

Dans l'exemple de réalisation des figures 13A et 13B, la zone utile 73 présente une section rétrécie par rapport à la zone de liaison 70. Ceci permet de créer un épaulement 76 faisant par exemple office de butée pour la bague d'embout 9 lorsque celle-ci est dans la position tirée, découvrant la buse 7, comme le montre par exemple le détail A de la figure 3. Une section plus faible permet aussi de former un trou plus petit dans le tissu lorsque la buse pénètre dans une muqueuse pour injecter du fluide, et cela permet aussi d'introduire plus facilement la buse 7 dans le tissu.

Dans l'exemple de réalisation décrit ici, le cathéter 1 est un cathéter bi-fonctionnel, c'est-à-dire que la buse 7 fait également office d'électrode. Par conséquent, la buse 7 est par exemple réalisée en un matériau conducteur, par exemple un métal, et est reliée par un fil conducteur 10, par exemple métallique, courant dans un canal 30 de la gaine intérieure 3, à une fiche électrique 14 reliée à une connexion électrique 51 du connecteur 5. Le fil conducteur 10 est de préférence soudé à une extrémité proximale 74b de la buse 7.

Dans le cas d'une buse faisant également office d'électrode, il est par exemple utile que l'extrémité distale 74a soit munie d'un anneau 78 (représenté figure 14). Pour assurer un meilleur positionnement de l'anneau 78, il est alors avantageux que l'extrémité distale 74a de la buse 7 présente un rétrécissement 77 (représenté ici sur le mode de réalisation de la figure 13), formant un autre épaulement, destiné à recevoir l'anneau 78. Un tel rétrécissement 77 pourrait aussi être formé à l'extrémité distale 74a de la buse 7 de la figure 12. L'anneau 78 peut cependant être fixé à l'extrémité distale 74a de la buse 7 sans un tel rétrécissement, par exemple par collage, mais cette configuration rend alors son positionnement plus délicat.

L'anneau 78 présente ici une forme de plateau circulaire troué de sorte à être ajusté à la section de l'extrémité distale 74a (i.e. de la zone utile 73 ou du rétrécissement 77 s'il existe).

D'un point de vue interne, comme le montre la figure 13B, la buse 7 comprend un canal 79 permettant d'acheminer un fluide depuis la gaine intérieure 3 à l'orifice de sortie 75. Dans l'exemple de réalisation de la figure 13B, le canal 79 présente une section qui, en moyenne, rétrécit en direction de l'orifice de sortie 75.

Plus précisément, le canal 79, relativement centré dans la buse 7, présente ici un tronçon 79a le plus large possible directement relié au canal 30 de la gaine intérieure 3, un tronçon en entonnoir 79b, un tronçon intermédiaire 79c qui est ici légèrement plus large que le tronçon en entonnoir 79b, et un tronçon d'injection 79d. Ces différents tronçons sont agencés de sorte à minimiser les turbulences dans l'écoulement du fluide tout en conservant une pression du fluide en sortie de l'orifice de sortie 75 avec un minimum de perte. On remarque par exemple que les tronçons 79a, 79b et 79c sont agencés de sorte à former une tuyère.

A l'autre extrémité de la gaine intérieure 3, à son extrémité proximale, la gaine intérieure 3 est fixée au tube 17, détaillé figure 6.

Le tube 17 est à son tour enfoncé dans une branche de sortie 53 du connecteur 5.

Dans le présent exemple de réalisation, comme détaillé figure 6, le tube 17, de forme globalement cylindrique, présente une gorge 170 s'étendant ici sur une majeure partie en longueur du tube 17. La gorge 170 permet par exemple de servir de déversoir pour de la colle si l'assemblage est réalisé par collage, afin d'éviter que de la colle ne déborde, ce qui procure en outre un rendu peu soigné et inesthétique. La gorge 170 permet aussi de favoriser l'agrippement du tube 17 dans le connecteur 5, par exemple assemblés par collage.

Comme mentionné précédemment, le connecteur 5 présente ici une branche de sortie 53. Il présente aussi au moins une branche 52 servant pour une connexion fluidique, désignée « connexion fluidique 52 », et dans le cas d'un cathéter bi-fonctionnel, il présente en outre une branche 51 servant pour une connexion électrique, désignée « connexion électrique 51 ». Ainsi, le connecteur 5 est ici un connecteur en Y, avec une connexion fluidique 52 pour le réservoir de fluide 6, une connexion électrique 51 et une connexion de sortie 53 le reliant à la gaine intérieure 3.

Le connecteur 5 présente un canal 56 permettant l'acheminement du fluide, qui est par conséquent en communication directe avec le canal 30 de la gaine intérieure 3.

Pour faciliter l'introduction du tube 17 dans la branche de sortie 53, la branche de sortie 53 présente ici une extrémité biseauté 54. Elle présente en outre un épaulement incliné 55 formant une butée pour le tube 17.

La connexion fluidique 52 présente ici une extrémité filetée 52a pour une connexion Luer-Lock pour une arrivée de fluide sous pression dans le canal 56, dont un tronçon 56a, présent au niveau de la branche 52, est élargi vers l'arrivée du fluide, par exemple pour limiter des turbulences dans l'écoulement lorsqu'il arrive dans le cathéter 1.

La branche 51 servant pour la connexion électrique reçoit la fiche électrique 14 reliée au fil conducteur 10.

La fiche électrique 14 est ici protégée par le protège fiche 18, qui est par exemple bloqué par son pied 180 entre les branches 51 et 52 du connecteur 5 d'une part, et le boitier 11 d'autre part lorsque l'ensemble du cathéter 1 est assemblé. La fiche électrique 14 est, quant à elle, par exemple bloquée en position par un épaississement 140 pris entre la branche 51 et un bord interne 181 du protège fiche 18.

Ainsi, le canal 56 du connecteur 5, le canal 30 de la gaine intérieure 3, et le canal 79 de la buse 7 permettent d'acheminer le fluide à injecter depuis un réservoir, comme par exemple le réservoir 6 d'un générateur de jets pulsés 101, jusqu'à l'orifice de sortie 75 de la buse 7.

Parallèlement, le fil conducteur 10 parcourt le canal 30 de la gaine intérieure 3 et le canal 56 du connecteur 5 en bifurquant dans la branche 51.

Une fois assemblés, les éléments précités sont alors fixes dans le boitier 11.

Par comparaison, le second ensemble est considéré comme mobile par rapport à ce premier ensemble.

La bague d'embout 9 (détaillée figure 11) est fixée dans une extrémité distale de la gaine extérieure 4, comme le montre le détail A de la figure 3. La bague d'embout 9 présente ici elle aussi une gorge 91 favorisant un meilleur agrippement dans la gaine extérieure 4 et permettant aussi de servir de déversoir pour de la colle si l'assemblage est réalisé par collage, afin d'éviter que de la colle ne déborde, ce qui procure en outre un rendu peu soigné et inesthétique. Comme mentionné précédemment, la bague d'embout 9 présente une forme distale arrondie formant une tête 90 de la bague d'embout 9. De préférence, la tête 90 de la bague d'embout 9 est un peu plus large de sorte à former une continuité avec une surface externe de la gaine extérieure 4 lorsqu'elles sont assemblées. La bague d'embout 9 comprend enfin un canal 93 terminé par un orifice 92 formé à un sommet de la tête 90. Le canal 93 présente par exemple un diamètre sensiblement égal à un diamètre de la zone utile 73 d'une buse 7 pour coulisser sur cette zone utile 73 tout en permettant une étanchéité entre la buse 7 et la bague d'embout 9 pour éviter que du fluide ou autre ne s'infiltre entre la gaine intérieure 3 et la gaine extérieure 4.

De plus, en position tirée (« a ») la bague d'embout 9 se retrouve par exemple en butée contre une extrémité distale de la gaine intérieure 3, voire aussi de l'épaulement 76 s'il existe, et en position poussée (« b ») la bague d'embout 9 se retrouve par exemple en butée par sa tête 90 contre l'anneau 78 si la buse 7 en est munie.

A une autre extrémité, son extrémité proximale, la gaine extérieure 4 est fixée dans l'élément intermédiaire 15 par la bague 16, comme le montre le détail B de la figure 3.

L'élément intermédiaire 15 est ici un élément élancé représenté schématiquement sur les figures 2 et 3, et détaillé figure 7.

La gaine extérieure 4 passe ici à l'intérieur de l'élément intermédiaire 15, et autour d'un corps 160 de la bague 16. Pour que la bague 16 puisse être insérée plus facilement dans la gaine extérieure 4, elle présente par exemple une première extrémité 161 biseauté. De plus, la bague 16 permet aussi de former une butée lorsque l'ensemble mobile est en position tirée (« a ») en venant en appui par exemple sur un renfort 130 du boitier 11. Pour cela, la bague 16 présente avantageusement une tête 162 plus large que le corps 160.

La tête 162 sert en outre de butée pour l'élément intermédiaire 15 lorsque la bague 16, entourée de la gaine extérieure 4, y est enfoncée. Enfin, la bague 16 présente un canal 163 de diamètre sensiblement égal à un diamètre de la gaine intérieure 3 pour pouvoir coulisser le long de la gaine intérieure 3.

L'élément intermédiaire 15 présente ici une première zone 151 destinée à coopérer avec le manchon 8, et une seconde zone 152 destinée à être positionnée à l'intérieur du boitier 11.

La première zone 151 présente un méplat 150, par exemple sur toute sa longueur, apte à coopérer avec un méplat 80 du manchon 8 pour induire par exemple un sens de montage obligatoire du manchon 8 sur l'élément intermédiaire 15. Ceci est par exemple utile si le manchon 8 comporte des inscriptions, comme par exemple des flèches illustrant les positions « a » et « b » pour sortir ou escamoter la buse 7.

La première zone 151 présente elle aussi des gorges 153 pour favoriser un agrippement avec le manchon 8. Les gorges 153 permettent aussi de servir de déversoir pour de la colle si l'assemblage est réalisé par collage, afin d'éviter que de la colle ne déborde, ce qui procure en outre un rendu peu soigné et inesthétique. La première zone 151 présente ici un diamètre globalement inférieur à un diamètre de la seconde zone 152 de sorte à former un épaulement 154 pouvant servir de butée à l'insertion du manchon 8. La première zone 151 présente enfin une zone de transition 155 permettant de réduire progressivement le diamètre de la première zone 151 par rapport à un diamètre de la gaine extérieure. Selon une option non représentée, une chemise souple, par exemple en élastomère, peut être présente entre l'élément intermédiaire 15 et le manchon 8 de sorte que le manchon 8 est monté en force, par exemple sur la chemise souple enfilée sur l'élément intermédiaire 15. La chemise souple peut par exemple s'étendre de part et d'autre au-delà de la première partie 151.

Une fois l'assemblage réalisé, le manchon 8 est par exemple en butée contre le boitier 11 lorsque le cathéter est en position tirée (« a »).

La seconde zone 152 présente en outre ici deux paires d'empreintes 157a, 157a' et 157b, 157b' aptes à coopérer avec deux plots 120, 120' formés dans le boitier 11. Les deux empreintes d'une paire (157a et 157a' d'une part, et 157b et 157b' d'autre part) sont de préférence formées de façon diamétralement opposée de sorte que les deux plots 120, 120' permettent aussi un maintien et un guidage de l'élément intermédiaire 15 dans le boitier 11. Toutefois, d'autres configurations peuvent être envisagées qui ne feraient par exemple appel qu'à une seule empreinte à la fois.

Les deux paires d'empreintes 157a, 157a' et 157b, 157b' permettent notamment ici un indexage de position tirée (« a ») ou poussée (« b »). En effet, en position tirée, la paire 157a, 157a' coopère avec les plots 120, 120', alors qu'en position poussée, c'est la paire 157b, 157b' qui coopère avec les plots 120, 120'. Lors du passage d'une position à l'autre, l'élément intermédiaire 15 est alors légèrement comprimé entre les plots 120, 120' ce qui donne à l'utilisateur une légère sensation de résistance jusqu'à ce que l'une des deux paires d'empreintes soit en vis-à-vis des plots 120, 120'. De plus, la mise en position des paires d'empreintes par rapport aux plots 120, 120' est sécurisée grâce aux différentes butées présentées précédemment (comme par exemple entre la bague d'embout 9 et l'anneau 78 d'une part ou la gaine intérieure 3 d'autre part, ou la bague 16 contre le renfort 130, ou encore le manchon 8 contre le boitier 11) qui empêchent un utilisateur de tirer ou pousser plus que nécessaire, ce qui pourrait par exemple abîmer les différents collages ou assemblage des pièces, ou encore les pièces elles-mêmes.

De plus, l'élément intermédiaire comprend aussi ici une rigole 156. La rigole 156 présente par exemple une longueur égale ou légèrement supérieure à un écart entre les deux paires d'empreintes 157a, 157a' et 157b, 157b'. La rigole 156 coopère ici avec un doigt 121 du boitier 11. La coopération entre la rigole 156 et le doigt 121 permet non seulement de sécuriser les mouvements de l'ensemble mobile pour éviter qu'un utilisateur ne tire au-delà de la position tirée ou ne pousse au-delà de la position poussée, mais aussi d'empêcher une rotation, voire une torsion, de l'ensemble mobile par rapport à l'ensemble fixe. De préférence, le doigt 121 présente alors un diamètre sensiblement égal à une largeur de la rigole 156. Dans le présent exemple de réalisation, la rigole 156 est positionnée entre les deux plots d'une même paire et à égales distances entre eux. Le doigt 121 est ici légèrement décalé par rapport aux plots 120, 120' comme le montre les figures 2 et 5. Ainsi, en position tirée (« a »), la paire d'empreinte 157a, 157a' coopère avec les plots 120, 120' tandis que le doigt 121 est proche, voire au contact pour former une butée, d'un fond 156a de la rigole 156. En position poussée (« b »), non représentée en détail ici, la paire d'empreinte 157b, 157b' coopèrerait avec les plots 120, 120' tandis que le doigt 121 serait proche, voire au contact pour former une butée, d'un fond 156b de la rigole 156.

Même si les différents éléments ne sont pas représentés à une même échelle, on constate cependant que l'écart entre la paire 157a, 157a', et la paire 157b, 157b' est bien supérieure à une longueur d'une partie de la zone utile 73 de la buse 7 qui peut être découverte ou escamotée. Ceci est notamment dû à un effet de structure (existence de jeux, élasticité du montage), ainsi qu'aux propriétés élastiques des différentes pièces et de leurs matériaux constitutifs. On rappelle par exemple que le conduit 2 mesure typiquement plus d'un mètre de long pour un diamètre maximal d'environ 2,5 mm, voire moins.

Dans le présent exemple de réalisation illustré ici, le boitier 11 est formé d'une partie mâle 12 (représenté figure 5) et d'une partie femelle 13 (représentée figure 4). Les plots 120, 120' sont ici formés dans la partie mâle 12. Sur la figure 3, illustrant le positionnement des différents composants du cathéter 1 dans la partie femelle 13 du boitier 11, les plots 120 et 120' ont toutefois été représentés en pointillés pour illustrer leur positionnement par rapport aux paires d'empreinte 157a, 157a' et 157b, 157b'.

Une fois les différents composants du cathéter 1 positionnés par exemple dans la partie femelle 13, le boitier 11 peut être refermé par la partie mâle 12, par exemple par soudage ou par collage. Le boitier 11 est par exemple réalisé en matière plastique, par exemple par moulage. Pour cet assemblage, la partie femelle 13 présente par exemple un rebord 13' et la partie mâle 12 présente par exemple un rebord 12' apte à s'accoler au rebord 13' dans la partie femelle 13.

Comme le montrent les figures 4 et 5, le boitier 11 comprend en outre différentes paires de pions d'assemblage 122, 132 (en l'occurrence six paires) comprenant un pion mâle 122 dans la partie mâle 12 et un pion femelle 132 dans la partie femelle 13 du boitier 11.

Le boitier comprend aussi des renforts, comme le renfort 130, coopérant avec un renfort 123 de la partie mâle. Les renforts 130 et 123 présentent respectivement une gorge 134 et 124 destinées à recevoir à la gaine intérieure 3 pour la maintenir en position. Les gorges 134 et 124 présentent par conséquent une courbure sensiblement égale à une courbure d'une section transversale de la gaine intérieure 3. La partie mâle présente en outre un renfort 125 muni d'une gorge 126, et la partie femelle présente un renfort 135 complémentaire au renfort 125, muni d'une gorge 136. Les gorges 126 et 136 présentent, quant à elles, une courbure sensiblement égale à une courbure d'une section transversale de la seconde zone 152 de l'élément intermédiaire 15 pour le maintenir en position tout en lui permettant de coulisser.

Enfin, le boitier comprend des découpes 127 et 137 pour le passage de la seconde zone 152 de l'élément intermédiaire 15 pour le maintenir en position tout en lui permettant de coulisser, des découpes 128, 138 pour le passage de la connexion fluide 52 du connecteur 5, et des découpes 129, 139 pour le passage de la connexion électrique 51 du connecteur 5 entourée du protège fiche 18.

La figure 17 présente de façon schématique un dispositif 100 selon un mode de réalisation de la présente invention.

Le dispositif 100 comprend au moins un générateur 101 et un cathéter 1 selon l'invention, par exemple tel que décrit précédemment en référence aux figures 1 à 16.

Un tel générateur est décrit de manière détaillée dans le document WO2011/148333 mais ses principales caractéristiques sont décrites ici.

Le générateur 101 est un générateur de jets pulsés haute pression prévu pour générer des impulsions de fluide sous haute pression à partir d'au moins un fluide contenu dans un réservoir formé ici d'une poche 6, souple, placée dans une enceinte 102, étanche, mise sous pression, par exemple grâce à une bouteille 103 contenant du gaz sous pression.

L'enceinte 102 présente au moins ici une sortie pour fluide 104, et une entrée de gaz sous pression 105.

Pour être mise sous pression, le générateur 101 comprend un dispositif de pressurisation pour l'enceinte 102 qui comprend une source de gaz sous pression, par exemple ici la bouteille 103 située hors de l'enceinte 102, qui fournit un gaz sous pression pénétrant dans l'enceinte 102 par l'entrée de gaz sous pression 105 pour augmenter pression interne de l'enceinte 102. Un dispositif de régulation de pression 106 permet de réguler la pression à l'intérieur de l'enceinte 102. Pour cela, le dispositif de régulation de la pression 106 est disposé à l'entrée de gaz sous pression 105, juste en amont, par exemple à l'extérieur de l'enceinte 102.

Le générateur 101 comprend par ailleurs un circuit fluidique qui comprend la poche 6 qui présente une sortie pour le fluide qu'elle contient, un connecteur de jonction 107, et un conduit de raccordement 108 lui-même relié à la poche 6 par le connecteur de jonction 107. Le connecteur de jonction 107, le conduit de raccordement 108 ainsi que la poche 6 sont disposés dans l'enceinte 102.

Le circuit fluidique comprend de plus un séquenceur hydraulique 109 relié en entrée à au moins le conduit de raccordement 108, et en sortie à un conduit de sortie 110 relié à la sortie de fluide 104 de l'enceinte 102.

Le séquenceur hydraulique 109 comprend notamment :
- une enveloppe déformable souple (non représentée), formée dans une liaison hydraulique qui relie le conduit de raccordement 108 au conduit de sortie 110 à l'intérieur du séquenceur 109, le tout étant exposé à la pression régnant dans l'enceinte 102,
- au moins un obturateur hydraulique (non représenté) dans la liaison hydraulique, qui peut être situé en amont ou en aval de l'enveloppe déformable souple, voire un en amont et un autre en aval,
- et un moyen de commande (non représenté) des différents obturateurs hydrauliques.

Le conduit de sortie 110 est à son tour relié à un conduit de liaison externe 112 avec le cathéter 1.

Pour garantir l'étanchéité au niveau de la sortie de fluide 104 de l'enceinte 102, la sortie de fluide 104 est de préférence munie d'un raccord à cône 111 par exemple, dont une partie effilée est de préférence dirigée vers l'extérieur de l'enceinte 102 de sorte que la pression interne de l'enceinte 102 tende à enfoncer le cône lorsque la pression croit.

Dans le présent exemple de réalisation, le séquenceur hydraulique 109 avec ses moyens de commande associés sont avantageusement compris dans une cassette remplaçable et interchangeable, ici placée à l'intérieur de l'enceinte 102.

Selon le présent exemple de réalisation, le générateur 1 01 est apte à générer un pulse de fluide à une pression comprise entre 0,2 et 3,9 MPa (2 et 30 bars), et d'un volume de fluide par pulse compris entre 100 µL- et 10 mL. Pour cela, il est alors préférable que l'enveloppe déformable souple présente un volume correspondant.

Bien entendu, plusieurs poches 6 pourraient être placées dans l'enceinte 102 si nécessaire, toutes reliées au séquenceur 109.

Le cathéter 1 est ainsi relié d'une part au générateur de jets haute pression 1 01 par la connexion fluidique 52 du connecteur 5. Dans le cas d'un cathéter bi-fonctionnel tel que décrit précédemment, le dispositif 100 comprend 20 en outre un générateur électrique 200 auquel le cathéter 1 est alors relié d'autre part, par exemple par un câble 201 raccordé à la connexion électrique 51 du connecteur 5. Le générateur électrique 200 est généralement désigné par le terme « ESU ».

Bien sûr, la présente invention ne se limite pas à la description précédente, mais s'étend à toute variante dans le cadre des revendications ci-après.

## Revendications

1. Cathéter (1) comprenant :
une gaine intérieure (3) comprenant un canal (30) ;
une buse d'injection (7) fixée à une extrémité distale de la gaine intérieure (3), la buse d'injection (7) étant réalisée en un matériau conducteur , la buse d'injection (7) comprenant une zone utile (73), dans laquelle la zone utile (73) présente une extrémité distale (74a) présentant un orifice de sortie (75) par lequel est injecté un fluide dans ou sous un tissu ;
une gaine extérieure (4) entourant la gaine intérieure (3) et coulissant par rapport à la gaine intérieure (3) ;
une bague d'embout (9) étant fixée à une extrémité distale de la gaine extérieure (4) et apte à coulisser sur au moins une partie de la buse d'injection (7) ;
dans lequel, dans une position poussée, la gaine extérieure (4) recouvre au moins une partie de la buse d'injection (7) et
dans une position tirée, la gaine extérieure (4) découvre ladite portion de la buse d'injection (7).

2. Cathéter (1) selon la revendication 1, comprenant en outre un anneau (78) fixé à l'extrémité distale (74a), l'anneau (78) ayant une forme de plateau circulaire troué de sorte à être ajusté à une section de l'extrémité distale (74a).

3. Cathéter (1) selon l'une quelconque des revendication 1 ou 2, dans lequel le bague d'embout (9) présente une forme distale arrondie formant une tête (90) de forme distale arrondie.

4. Cathéter (1) selon l'une quelconque des revendications précédentes, dans lequel en position poussée, la bague d'embout (9) se retrouve en butée par sa tête (90) contre l'anneau (78).

5. Cathéter (1) selon l'une quelconque des revendications précédentes, dans lequel en position tirée, la bague d'embout (9) se retrouve en butée contre un épaulement (76) de la buse d'injection (7).

6. Cathéter (1) selon l'une quelconque des revendications précédentes, dans lequel la buse d'injection (7) comprend un canal (79), le canal (30) de la gaine intérieure (3) et le canal (79) de la buse d'injection (7) permettent d'acheminer un fluide à injecter jusqu'à l'orifice de sortie (75).

7. Cathéter (1) selon la revendication précédente, dans lequel le canal (79) de la buse d'injection (7) présente un tronçon (79a) le plus large possible directement relié au canal (30) de la gaine intérieure (3), un tronçon en entonnoir (79b), un tronçon intermédiaire (79c) qui est plus large que le tronçon en entonnoir (79b), et un tronçon d'injection (79d).

8. Cathéter (1) selon l'une quelconque des revendications précédentes, dans lequel la buse d'injection (7) est reliée à une connexion électrique (51) par un fil conducteur (10) ,
la bague d'embout (9) est en céramique ,
la gaine extérieure (4) est réalisée en téflon ,
la buse d'injection (7) comprend une zone de liaison (70) présentant des gorges (71) permettent un meilleur agrippement entre la buse d'injection (7) et la gaine intérieure (3).

9. Cathéter (1) selon la revendication précédente, dans lequel la zone utile (73) présente une section rétrécie par rapport à la zone de liaison (70).

10. Cathéter (1) selon l'une quelconque des revendications précédentes, comprenant en outre un boitier (11) comprenant une découpe (128, 138) pour un passage d'une connexion fluide (52) et une découpe (129, 139) pour un passage d'une connexion électrique (51).

11. Cathéter (1) selon la revendication précédente, comprenant en outre un manchon (8) fixé à la gaine extérieure (4), le manchon étant apte à coulisser par rapport au boitier (11) , dans lequel la buse d'injection (7) est apte à être sortie par une traction de la gaine extérieure (4), via le manchon (8), permettant d'ajuster sa position.

## Patentansprüche

1. Katheter (1) aufweisend:
eine innere Hülle (3) mit einem Kanal (30);
eine Einspritzdüse (7), die an einem distalen Ende der inneren Hülle (3) angebracht ist, wobei die Einspritzdüse (7) aus einem elektrisch leitfähigen Material hergestellt ist, die Einspritzdüse (7) eine Anwendungszone (73) hat, die Anwendungszone (73) ein distales Ende (74a) mit einer Austrittsöffnung hat, via welche ein Fluid in oder unter ein Gewebe injiziert wird;
eine äußere Hülle (4), der die innere Hülle (3) umgibt und bezüglich der inneren Hülle (3) gleitend ist;
einen Aufsatzring (9), der an einem distalen Ende der äußeren Hülle (4) angebracht ist und fähig ist, über mindestens einen Teil der Einspritzdüse (7) zu gleiten;
wobei in einer vorgeschobenen Position die äußere Hülle (4) mindestens einen Teil der Einspritzdüse (7) bedeckt, und
in einer zurückgezogenen Position die äußere Hülle (4) den Teil der Einspritzdüse (7) nicht bedeckt.

2. Katheter (1) nach Anspruch 1, aufweisend ferner einen an dem distalen Ende (74a) angebrachten Ring (78), wobei der Ring (78) in Form einer kreisrunden Platte ist, die derart gelocht ist, um an einen Querschnitt des distalen Endes (74a) angepasst zu sein.

3. Katheter (1) nach einem der Ansprüche 1 oder 2, wobei der Aufsatzring (9) eine abgerundete distale Form hat, die einen Kopf (90) mit distaler abgerundeter Form bildet.

4. Katheter (1) nach einem der vorstehenden Ansprüche, wobei in der vorgeschobenen Position der Aufsatzring (9) mit seinem Kopf (90) im Anschlag gegen den Ring (78) angeordnet ist.

5. Katheter (1) nach einem der vorstehenden Ansprüche, wobei in der zurückgezogenen Position der Aufsatzring (9) im Anschlag gegen eine Schulter (76) der Einspritzdüse (7) angeordnet ist.

6. Katheter (1) nach einem der vorstehenden Ansprüche, wobei die Einspritzdüse (7) einen Kanal (79) aufweist, der Kanal (30) der inneren Hülle (3) und der Kanal (79) der Einspritzdüse (7) erlauben, ein zu injizierendes Fluid zu der Austrittsöffnung (75) zu leiten.

7. Katheter (1) nach dem vorstehenden Anspruch, wobei der Kanal (79) der Einspritzdüse (7) einen möglichst weiten Abschnitt (79a), der direkt mit dem Kanal (30) der inneren Hülle (3) verbunden ist, einen trichterförmigen Abschnitt (79b), einen Zwischenabschnitt (79c), der weiter als der trichterförmige Abschnitt (79b) ist, und einen Injektionsabschnitt (79d) aufweist.

8. Katheter (1) nach einem der vorstehenden Ansprüche, wobei die Einspritzdüse (7) via einen Leitungsdraht (10) mit einem elektrischen Anschluss (51) verbunden ist,
der Aufsatzring (9) aus Keramik ist,
die äußere Hülle (5) aus Teflon hergestellt ist,
die Einspritzdüse (7) eine Kopplungszone (70) mit Auskehlungen (71) aufweist, die einen besseren Eingriff zwischen der Einspritzdüse (7) und der inneren Hülle (3) erlauben.

9. Katheter (1) nach dem vorstehenden Anspruch, wobei die Anwendungszone (73) einen bezüglich der Kopplungszone (70) engeren Querschnitt hat.

10. Katheter (1) nach einem der vorstehenden Ansprüche, aufweisend ferner ein Gehäuse (11), das eine Aussparung (128, 138) für einen Durchlass eines Fluidanschlusses (52) und eine Aussparung (129, 139) für einen Durchlass eines elektrischen Anschlusses (51) aufweist.

11. Katheter (1) nach dem vorstehenden Anspruch, aufweisend ferner eine an der äußeren Hülle (4) angebrachte Muffe (8), wobei die Muffe fähig ist, bezüglich des Gehäuses (11) zu gleiten, wobei durch Ziehen der äußeren Hülle (4) via die Muffe (8) die Einspritzdüse (7) ausgefahren werden kann, um ein Anpassen ihrer Position zu erlauben.

## Claims

1. A catheter (1) comprising:
an inner sheath (3) comprising a canal (30);
an injection nozzle (7) attached to a distal end of the inner sheath (3), the injection nozzle (7) being made of a conductive material, the injection nozzle (7) comprising an operating area (73), wherein the operating area (73) has a distal end (74a) with an outlet orifice (75) through which a fluid is injected into or under a tissue;
an outer sheath (4) surrounding the inner sheath (3) and sliding relative to the inner sheath (3);
a tip ring (9) fastened to a distal end of the outer sheath (4) and adapted to slide over at least part of the injection nozzle (7);
wherein, in a pushed position, the outer sheath (4) covers at least part of the injection nozzle (7), and
in a retracted position, the outer sheath (4) exposes said portion of the injection nozzle (7).

2. The catheter (1) according to claim 1, further comprising an annulus (78) fastened to the distal end (74a), the annulus (78) having the shape of a circular plate with holes so as to fit a section of the distal end (74a).

3. The catheter (1) according to any one of claims 1 or 2, wherein the tip ring (9) has a rounded distal shape forming a head (90) of rounded distal shape.

4. The catheter (1) according to any one of the preceding claims, wherein, in the pushed position, the tip ring (9) abuts against the annulus (78) with its head (90).

5. The catheter (1) according to any one of the preceding claims, wherein, in the pulled position, the tip ring (9) abuts against a shoulder (76) of the injection nozzle (7).

6. The catheter (1) according to any one of the preceding claims, wherein the injection nozzle (7) comprises a channel (79), wherein the channel (30) of the inner sheath (3) and the channel (79) of the injection nozzle (7) enable a fluid to be injected to be delivered to the outlet orifice (75).

7. The catheter (1) according to the preceding claim, wherein the channel (79) of the injection nozzle (7) has a widest possible section (79a) directly connected to the channel (30) of the inner sheath (3), a funnel-shaped section (79b), an intermediate section (79c) that is wider than the funnel-shaped section (79b), and an injection section (79d).

8. The catheter (1) according to any one of the preceding claims, wherein the injection nozzle (7) is connected to an electrical connection (51) by a conductive wire (10),
the tip ring (9) is of ceramic,
the outer sheath (4) is made of Teflon,
the injection nozzle (7) comprises a connection area (70) having grooves (71) that enable a better grip between the injection nozzle (7) and the inner sheath (3).

9. The catheter (1) according to the preceding claim, wherein the operating area (73) has a section that is narrowed with regard to the connection area (70).

10. The catheter (1) according to any one of the preceding claims, further comprising a housing (11) having a cutout (128, 138) for the passage of a fluid connection (52) and a cutout (129, 139) for the passage of an electrical connection (51).

11. The catheter (1) according to the preceding claim, further comprising a sleeve (8) fixed to the outer sheath (4), the sleeve being slidable relative to the housing (11), wherein the injection nozzle (7) can be moved outwards by pulling on the outer sheath (4) via the sleeve (8), thereby enabling its position to be adjusted.
